# EUROPEAN PATENT SPECIFICATION

(11) **EP 0 582 295 B1**
(45) Date of publication and mention of the grant of the patent: **13.01.1999**
(21) Application number: 93112520.7
(22) Date of filing: 04.08.1993
(51) Int. Cl.: A61B 17/28, A61B 17/02

(54) **Articulating endoscopic surgical apparatus**
Endoskopisches chirurgisches Gelenkwerkzeug
Dispositif chirurgical endoscopique articulé

(30) Priority: 05.08.1992 US 925496
(43) Date of publication of application: 09.02.1994
(73) Proprietor: United States Surgical Corporation, Norwalk, Connecticut 06856 (US)
(72) Inventor: Nicholas, David A., Trumbull, CT 06611 (US); Aranyi, Ernest, Easton, CT 06612 (US); Zvenyatsky, Boris, Bronx, NY 10462 (US); Matula, Paul A., Brookfield, CT 06804 (US); Remiszewski, Stanley H., Greenwich, CT 06830 (US); Green, David T., Westport, CT 06880 (US); Bolanos, Henry, East Norwalk, CT 06855 (US)
(74) Representative: Marsh, Roy David

(56) References cited:
- EP-A- 0 165 472
- EP-A- 0 405 250
- EP-A- 0 412 282
- EP-A- 0 449 663
- EP-A- 0 484 671
- DE-A- 3 303 335
- DE-U- 8 535 164
- DE-U- 8 808 285
- DE-U- 9 300 161
- FR-A- 2 469 912
- US-A- 2 028 635
- US-A- 3 144 020
- US-A- 4 872 456
- US-A- 4 950 273
- US-A- 5 131 382

## Description

### CROSS-REFERENCE TO RELATED APPLICATION

Related to this application is Applicant's earlier EP-A-557806. Indeed Figures 1 to 21 of the drawings are common to the two patent applications.

### BACKGROUND OF THE INVENTION

### 1. Field of the Invention

The subject invention relates to surgical apparatus for performing laparoscopic and endoscopic surgical procedures, and more particularly to apparatus having an end portion which can be articulated in a patient's body during a surgical procedure. Such an apparatus is disclosed in US-A-4880015 which shows all the technical features of the pre-characterising part of claim 1 below.

### 2. Description of Related Art

In laparoscopic and endoscopic surgical procedures a small incision or puncture is made in the patient's body, the cannula allows insertion of various surgical instruments such as scissors, dissectors or retractors to perform the surgery.

An example of an endoscopic surgical instrument is illustrated in U.S. Patent No. 2,113,246 which issued to Wappler on April 5th, 1938. This patent discloses endoscopic forceps comprising an elongated conduit with jaws at the distal end thereof, a control rod in the conduit for controlling the operation of the jaws, and a control handle at the proximal end of the conduit which is operatively connected to the control rod. This surgical instrument is extremely limited in its applications in that the angle of the conduit portion mounting the jaws cannot be adjusted in relation to the remaining portion of the conduit during a surgical procedure.

Improvements have been made in the art of surgical instruments to increase their range of operability. For example, U.S. Patent No. 4,763,699 which issued to Jaeger on August 16, 1988 discloses a microsurgery instrument with an adjustable angle of operation for obtaining cervical biopsies.

Similarly, U.S. Patent No. 4,880,015 which issued to Nierman on November 14, 1989 discloses a surgical device having an increased range of operability. In particular, this patent shows a biopsy forceps designed for use through a flexible fiberoptic bronchoscope. The biopsy forceps includes a handle connected to a thin elongated flexible shaft with a distal portion thereof hinged to the shaft. A grasping tool or biopsy forceps attached to the distal hinged portion. Control wires extend from the handle to the distal end of the shaft for controlling the angular rotation of the distal portion of the instrument. The two-part form of claim 1 is based upon US-A-4,880,015. Design considerations taken into account by the present inventors when they made the present invention included the following:
- to provide an endoscopic surgical instrument in which a wide variety of different tool heads may be employed,
- to provide an endoscopic surgical instrument which may be used to perform electrocauterization during surgical procedures,
- to provide an endoscopic surgical instrument which may be used for performing retraction, grasping or dissecting tasks during gynaecological procedures,
- to provide an endoscopic surgical instrument for performing retraction, grasping or dissecting tasks during abdominal surgery, and
- to provide endoscopic surgical instrument which is inexpensive to manufacture.

### SUMMARY OF THE INVENTION

The technical features which characterise the present invention are recited in claim 1 below.

An endoscopic surgical instrument is disclosed, with potential for use in a wide variety of roles including grasping, dissecting, clamping, or retracting materials or tissue during surgical procedures performed within a patient's body and particularly within the abdominal cavity.

Preferred embodiments of the inventive instrument of claim 1 below are defined in the dependent claims.

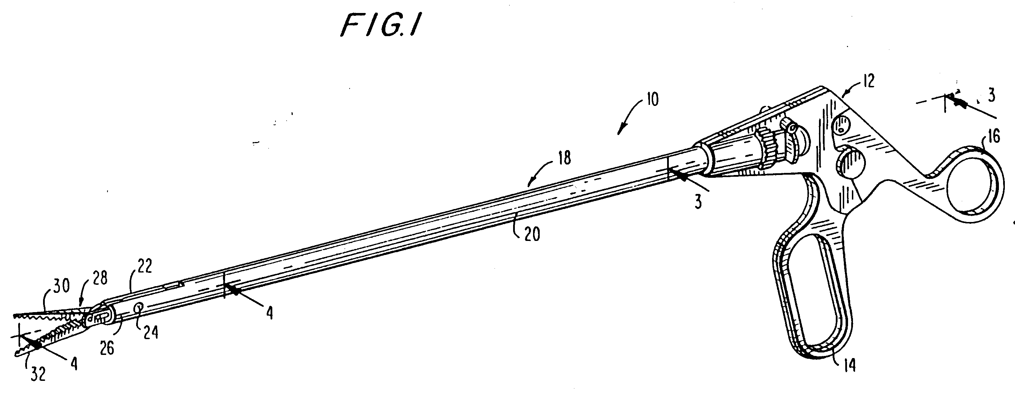

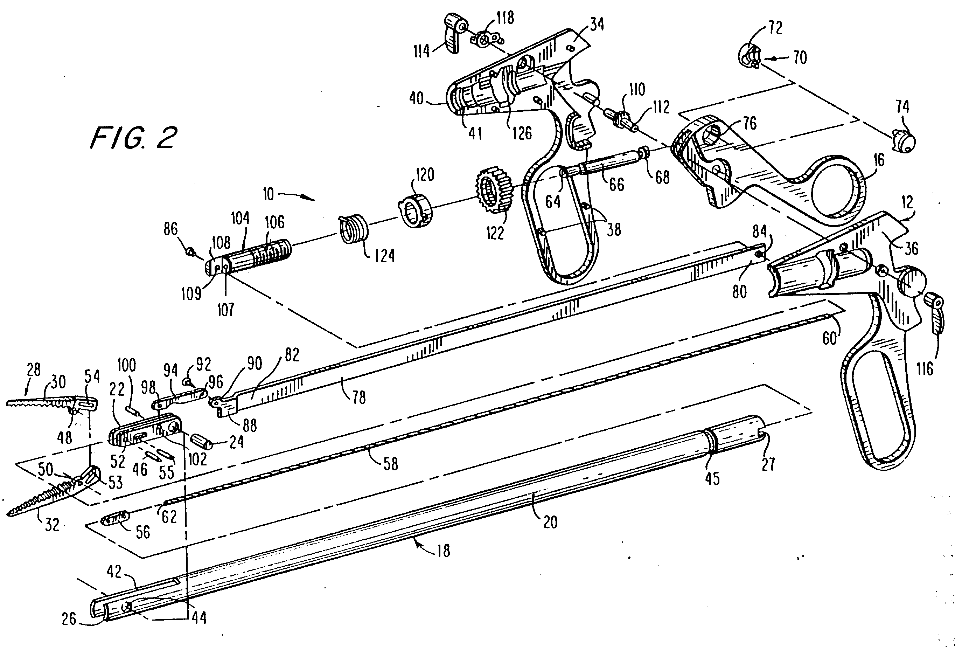

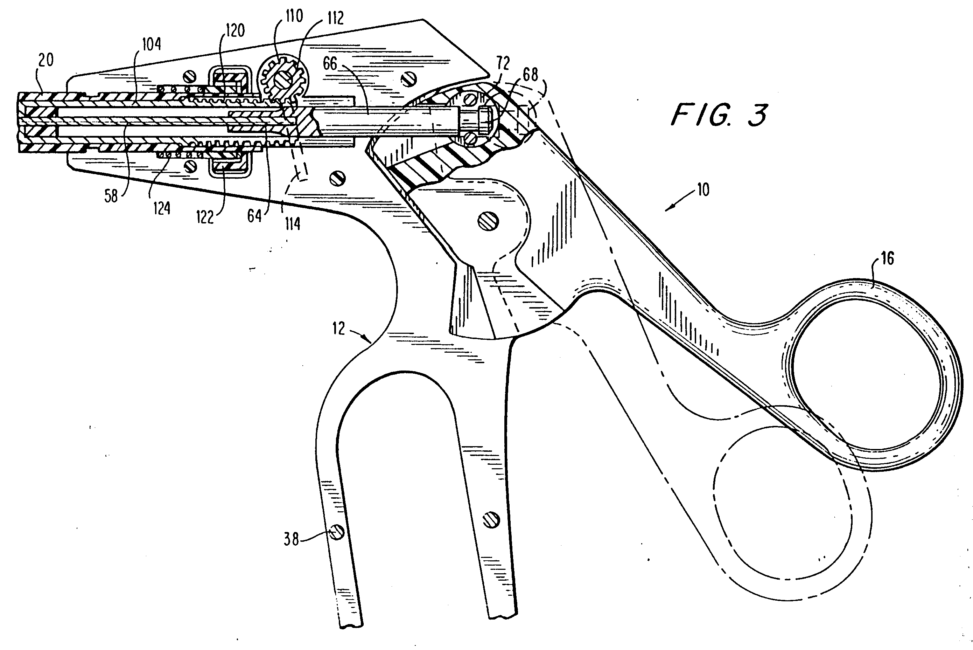

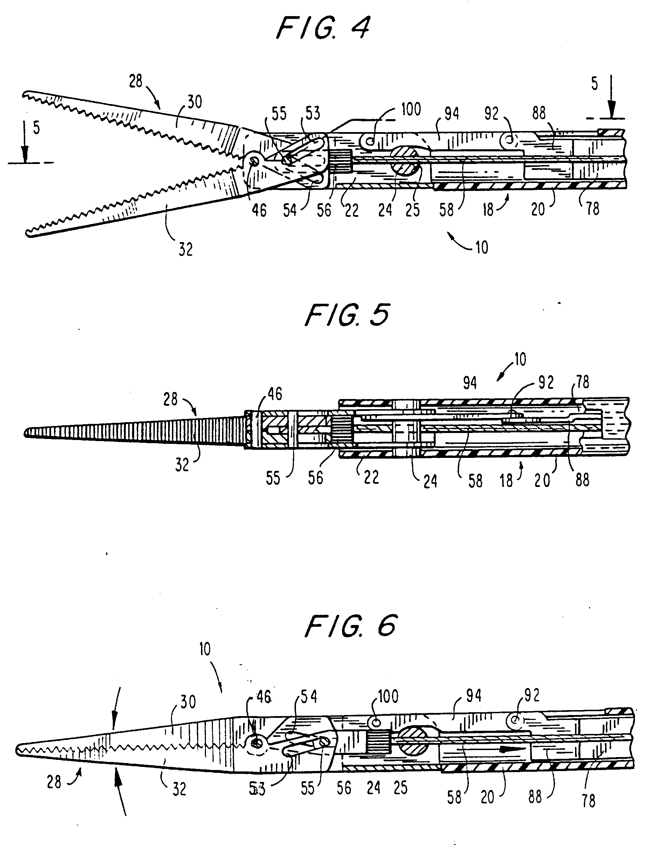

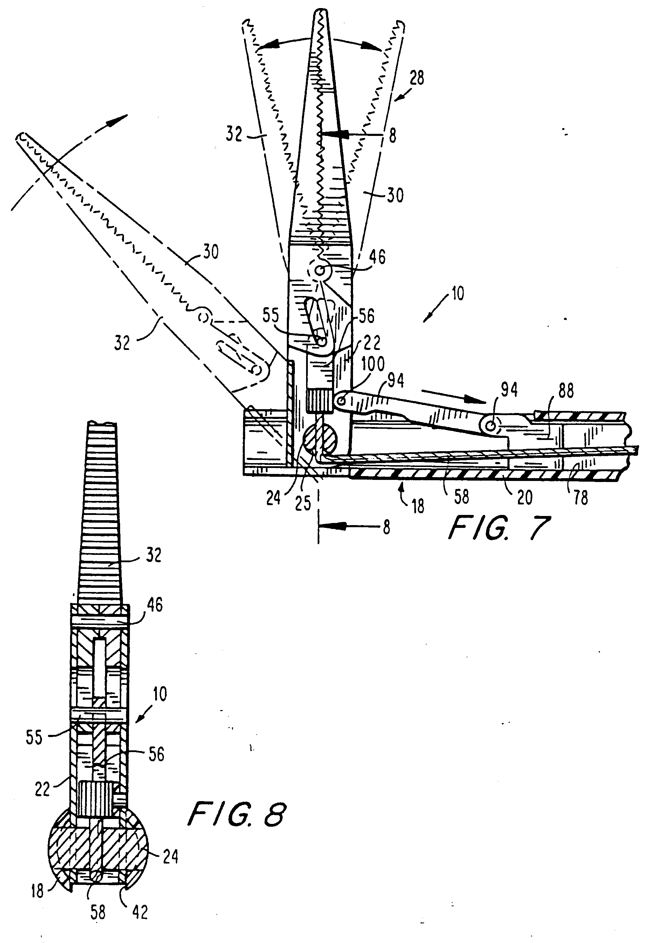

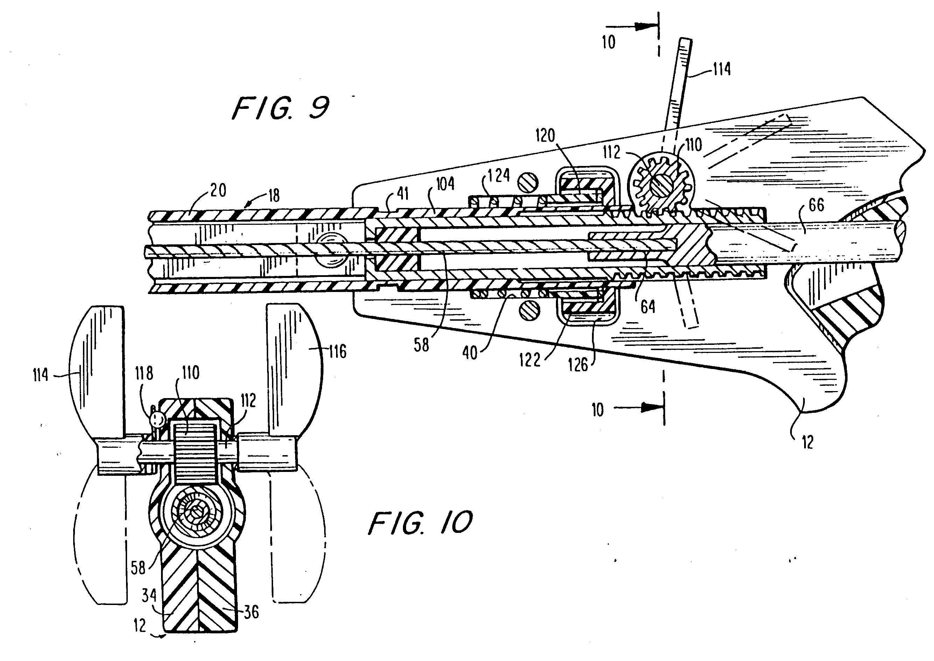

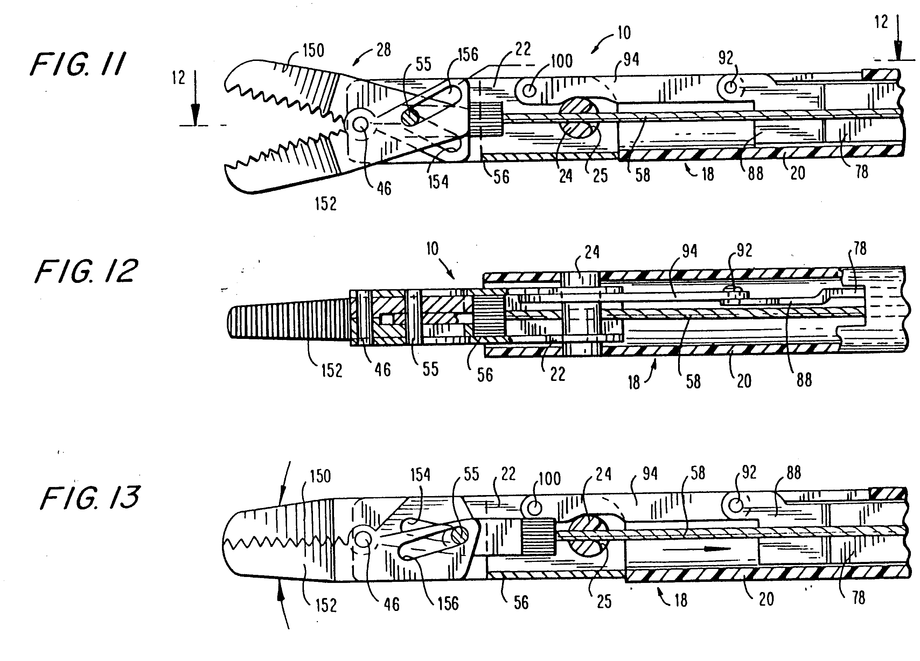

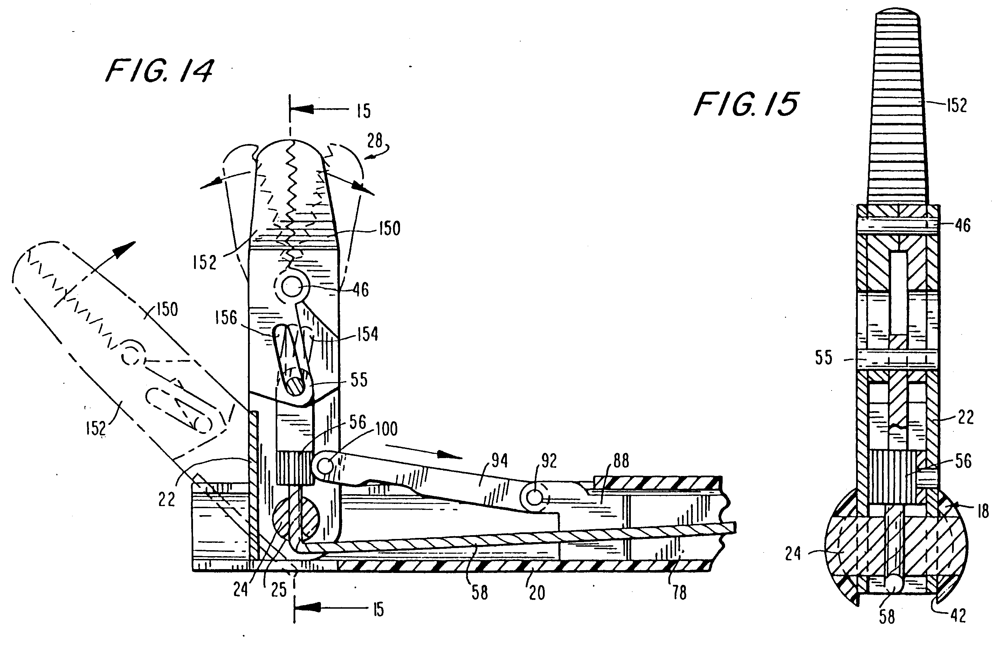

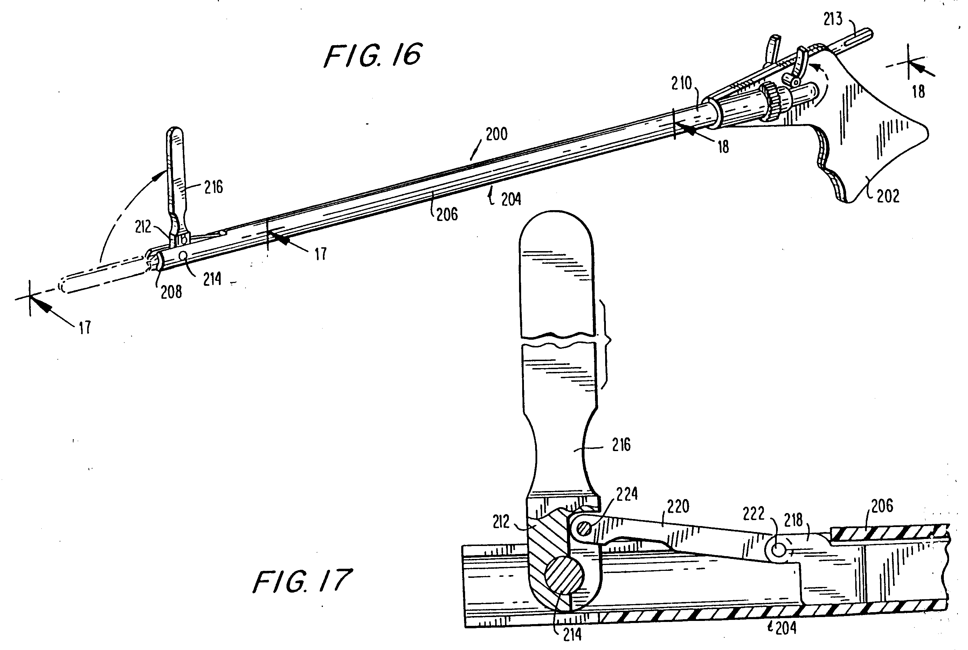

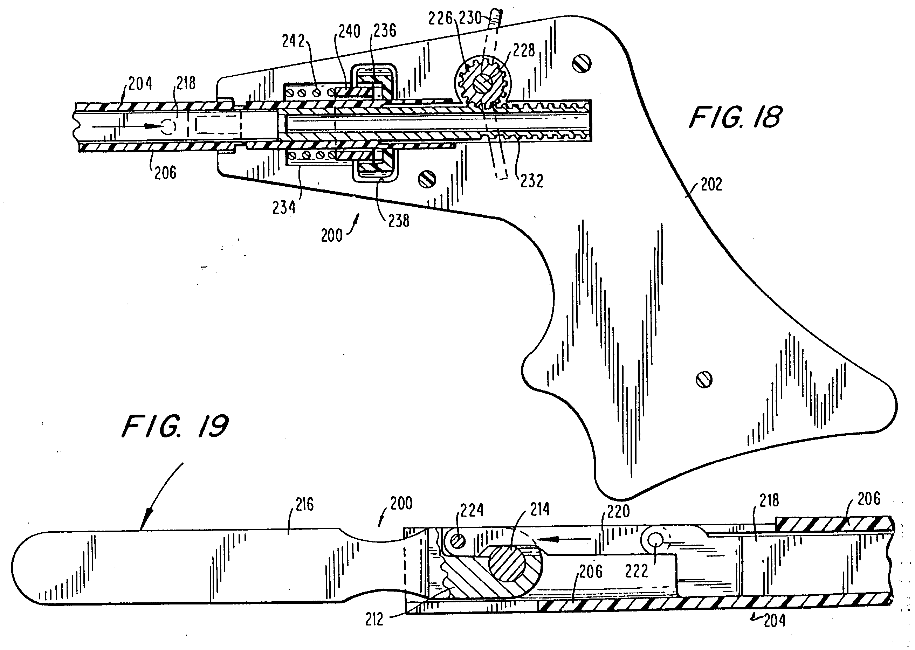

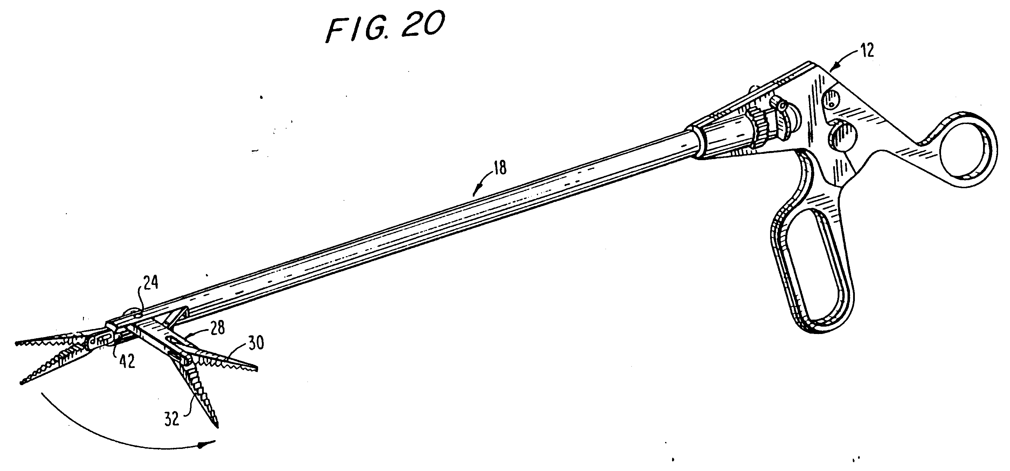

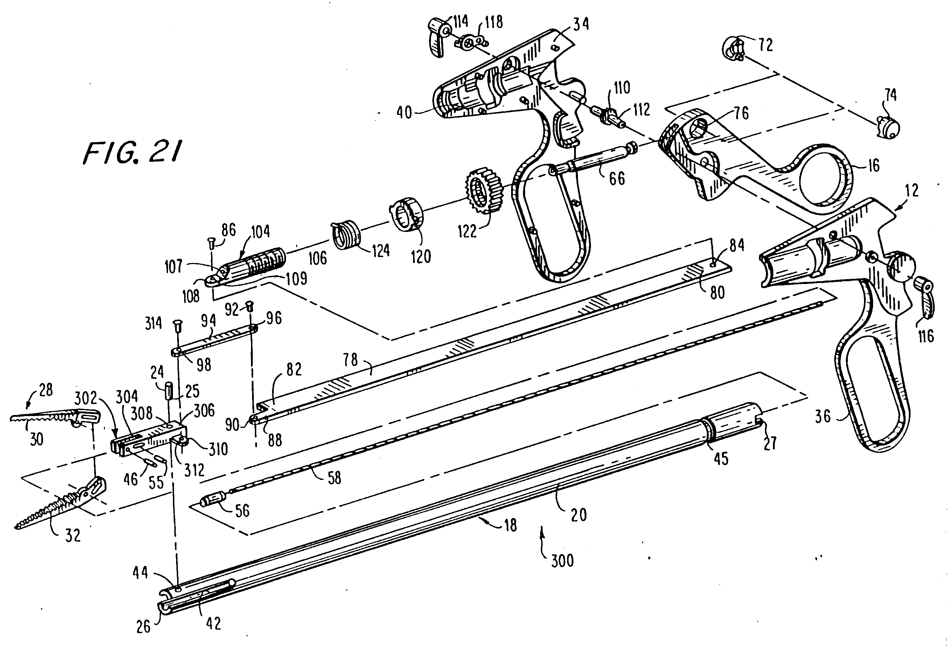

Further features of the subject invention will become more readily apparent from the following detailed description of the invention taken in conjunction with accompanying drawings. A detailed explanation of Figs. 1 to 21, showing tools which can be combined with the claimed apparatus and some features without relevance to the claimed subject matter, is omitted, because it can be studied in above-mentioned EP-A-557806.

### BRIEF DESCRIPTION OF THE DRAWINGS

Preferred embodiments of the subject invention will be described hereinbelow with reference to Figures 22 to 35 of the drawings. In the drawings:
Fig. 1 is a perspective view of an articulating endoscopic surgical instrument
Fig. 2 is an exploded view of the articulating endoscopic surgical instrument of Fig. 1;
Fig. 3 is a side cross-sectional view taken along line 3-3 of Fig. 1 illustrating the handle portion of the endoscopic instrument;
Fig. 4 is a side cross-sectional view taken along line 4-4 of Fig. 1 illustrating the distal end of the endoscopic surgical instrument;
Fig. 5 is a top plan view cross-section view taken along line 5-5 of Fig. 4;
Fig. 6 is a side cross-sectional view of the distal end of the endoscopic surgical instrument showing the jaws in a closed position;
Fig. 7 is a side cross-sectional view showing, in solid and in phantom lines, the various pivoting movements of the articulating section of the endoscopic portion of the surgical instrument shown in Fig. 1;
Fig. 8 is an enlarged side cross-sectional view of the distal end of the endoscopic surgical instrument taken along line 8-8 of Fig. 7;
Fig. 9 is an enlarged side cross-sectional view of the distal end of the endoscopic surgical instrument illustrating the various positions of the pinion gear which comprises a portion of the linkage mechanism;
Fig. 10 is a front cross-sectional view taken along line 10-10 of Fig. 9;
Fig. 11 is a side cross-sectional view illustrating an alternative embodiment of the tool head of the endoscopic surgical instrument in an open position.
Fig. 12 is a top cross-sectional view taken along line 12-12 of Fig. 11;
Fig. 13 is a side cross-sectional view of the tool head shown in Fig. 11 in a closed position.
Fig. 14 is a side cross-sectional view showing, in a solid and in phantom lines the pivotal movements of the articulating section of the endoscopic portion of the instrument with the alternate form of the tool head shown in Figs 11-13;
Fig. 15 is a front cross-sectional view taken along line 15-15 of Fig.
Fig. 16 is a perspective view of an alternate form of the instrument which includes an articulating paddle for performing retraction tasks;
Fig. 17 is a side cross-sectional view taken along line 17-17 of Fig. 16;
Fig. 18 is a side cross-sectional view taken along line 18-18 of Fig. 16;
Fig. 19 is a side cross-sectional view taken along line 17-17 of Fig. 16;
Fig. 20 is a perspective view of another form of the articulating endoscopic surgical instrument;
Fig. 21 is an exploded view of the articulating endoscopic surgical instrument of Fig. 20;
Fig. 22 is a perspective view of embodiment of the articulating endoscopic surgical instrument of the present invention adapted for gynaecological procedures;
Fig. 23 is an exploded perspective view of the articulating endoscopic surgical instrument of Fig. 22;
Fig. 24 is a side cross-sectional view of the endoscopic surgical instrument of Fig. 22 with the retractor assembly thereof in a closed position;
Fig. 25 is a side cross-sectional view of the endoscopic surgical instrument of Fig. 22 with the retractor assembly thereof in an open position;
Fig. 26 is a side cross-sectional view of the endoscopic surgical instrument of Fig. 22 with the distal end portion thereof disposed in an articulated position;
Fig. 27 is a perspective view of another embodiment of the endoscopic surgical instrument of the subject invention;
Fig. 28 is an exploded perspective view of the articulating endoscopic surgical instrument of Fig. 27;
Fig. 29 is a top cross-sectional view of the articulating endoscopic surgical instrument of Fig. 27 with tile retractor assembly thereof in a closed position;
Fig. 30 is a top cross-sectional view of the articulating endoscopic instrument of Fig. 27 with the retractor assembly thereof in an opened position;
Fig. 31 is a side cross-sectional view of the articulating endoscopic instrument of Fig. 27;
Fig. 32 is a side cross-sectional view of the articulating endoscopic surgical instrument of Fig. 27 with the distal end portion thereof disposed in an articulated position;
Fig. 33 is a side cross-sectional view of an endoscopic surgical instrument of the present invention equipped with a removable cervix seal; and
Figs. 34 and 35 are side cross-sectional views of an endoscopic surgical instrument of the present invention having fluid injection structure and an articulating sleeve cover.

For a detailed description of what is shown in Figs 1 to 21, the reader is referred to EP-A-557806. Although these figures do not show embodiments of the present invention nevertheless they illustrate closely related subject matter which helps, rather than hinders, a full understanding of the present invention

### DETAILED DESCRIPTION OF THE PREFERRED EMBODIMENT

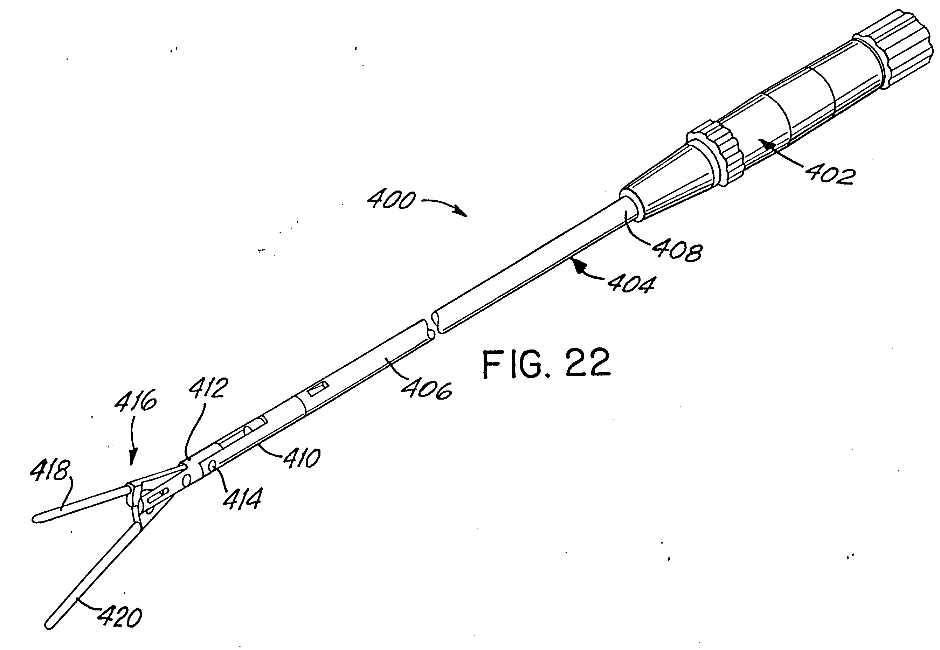

Referring to Fig. 22, an endoscopic surgical instrument 400 of the subject invention is illustrated which may be used as a retractor during gynecological procedures and particularly an intrauterine retractor. Surgical instrument 400 comprises a substantially barrel-shaped axial handle portion 402, and an elongated endoscopic portion 404 which extends outwardly from handle portion 402. Endoscopic portion 404 includes a tubular section 406 mounted by a pivot pin 414 adjacent the distal end portion 410 thereof. A retractor assembly 416 is operatively associated with the articulating section 412 of endoscopic portion 404 and includes a pair of co-operating atraumatic rod members 418 and 420 each having cylindrical bodies with blunt heads configured so as not to cause damage to tissue during retraction procedures.

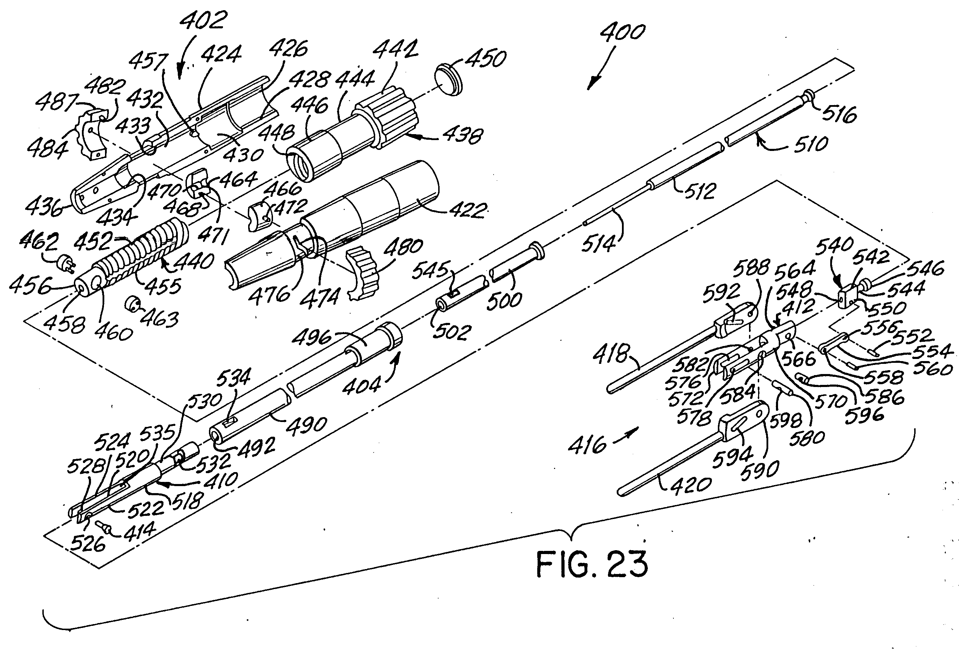

Turning to Fig. 23, the handle portion 402 of surgical retractor 400 includes a two-part handle having right and left mountable hemi-sections 422 and 424. Once assembled, the hemi-sections 422 and 424 defined a stepped axial bore 426 which extends through the handle portion 402. The axial bore 426 has a proximal chamber 428, a primary medical chamber 430, a secondary medial chamber 432, a tertiary medial chamber 433, a quaternary medial chamber 434, and a distal chamber 436 defined therein. The handle portion 402 houses, within the axial bore 426, a driving assembly which manipulates the retractor assembly 416, and a camming assembly which manipulates the articulating section 412 of endoscopic portion 404.

The driving assembly of surgical instrument 400 includes knob member 438 and an associated threadably advanceable driving screw member 440. Knob member 438 comprises a proximal grasping portion 442, an intermediate cavity portion 444, and a distal engaging portion 446. A threaded axial bore 448 extends at least partially through the knob member 438 from the distal engaging portion 446 thereof to the proximal grasping portion 442 where it is capped by a threaded closure member 450. Knob member 438 is mounted within the axial bore 426 of handle portion 402 in such a manner so that the distal engaging portion 446 thereof is maintained within the primary medial chamber 430 of axial bore 426 which the intermediate cavity portion 444 is maintained within the proximal chamber 428 of axial bore 426. Once mounted, knob member 438 is rotatable about the longitudinal axis of handle portion 402.

The driving screw member 440 of the driving assembly comprises an elongated threaded body portion 452 having a proximal end portion 454 which engages operatively within the threaded axial bore 448 of knob member 438, and a distal head portion 456 which extends from the threaded body potion 452 thereof. Lateral slots extend along the body portion 452 of screw member 440 for co-operatively engaging protuberances 457 projecting radially into the secondary medial chamber 432 of axial bore 426. The engagement of the protuberance 457 within lateral slot 455 prohibits rotational movement of screw member 440 when the knob member 438 is rotated and in addition, provides guidance for the screw member 440 within the axial bore 426.

A bore 458 extends axially into the distal head potion 456 of the driving screw member 440 for permitting retention of elements of endoscopic portion 404 therein. A transverse aperture 460 is provided in the head portion 456 of screw member 440 for accommodating a two-part universal locking clip 462, 463 which engages an element of endoscopic portion 404 extending into bore 458. Once mounted within the handle portion 402, the threaded body portion 452 of screw member 440 is maintained partially within the secondary medial chamber 432 of axial bore 426. Rotations of the knob member 438 relative to the handle portion 402 will cause corresponding axial translation of tile driving screw member 440 within the secondary medial chamber 432 of axial bore 426 in handle portion 402.

The camming assembly for manipulating the articulating section 412 of endoscopic portion 404 includes a cylindrical cam follower having right and left hemi-portions 464 and 466 mountable to one another by bosses such as, for example, boss 468 on right hemi-portion 464. An axial pathway 470 is formed in the cylindrical cam follower 464, 466 for accommodating elements of endoscopic portions 404. In particular, an annular groove 471 is formed therein for engagement purposes. A cam follower post 472 projects radially outward from the periphery of left hemi-portion 466 and travels within cam slot 474 formed in the left hemi-section 422 of handle portion 402. The cam slot 474 is disposed within a circumferential groove 476 defined in the outer surface of handle portion 402 external from the tertiary medial chamber 433 of axial bore 426.

The cylindrical cam follower 464, 466 is adapted and configured to move axially and rotatably within the tertiary medial chamber 433 of stepped axial bore 426 in response to rotation of a two-part manipulating collar having right and left hemi-portions 478 and 480 mountable to one another by mounting projections, such as, for example, mounting projection 482. An aperture 484 extends radially through hemi-portion 478 of the manipulating collar for receivably engaging the cam follower post 472 so as to operatively interlock the elements of the camming assembly. Rotation of collar 478, 480 will cause cam post 472 to be driven in a cam slot 474 causing axial advancement of the camming assembly relative to the handle portion 402.

The endoscopic portion 404 of surgical instrument 400 extends from handle portion 402, and comprises a plurality of coaxial tubular members including an outer tubular member 490 having an axial bore 492 and a stepped proximal end which defines a first annular ridge portion 494, and a second annular ridge portion 496 for mounting the endoscopic portion 404 to handle portion 402. The first annular ridge portion 494 is maintained in the quantenary medial chamber 434 of axial bore 426, while the second annular ridge portion 496 is maintained in the distal chamber 436 of axial bore 426 for mounting the endoscopic portion 402 to handle portion 402.

Endoscopic portion 404 also includes an inner tubular member 500 having an axial passageway 502, and a proximal annular mounting flange 504 dimensioned for locking engagement in the annular groove 471 of the axial bore 470 in cylindrical cam follower 464, 466. An internal control member 510 extends through the axial passageway 502 of inner tubular member 600 and includes an outer sleeve 512, and a central rod 514. Central control rod 514 has a proximal tail portion 516 which is engagable within the distal head portion 456 of screw member 440 by the two-part universal clip 462, 463 such that axial translation of screw member 440, in response to rotations of knob member 438, will cause corresponding axial translation of the center control rod 514 within endoscopic portion 404. A distal end portion 515 of central control rod 514 is flexible for permitting operation of the retractor assembly 416 when the articulating section 412 is pivoted into an operative position.

The distal end portion 410 of endoscopic portion 404 defines a sleeve member which includes a yoke portion 518 having a longitudinally extending slot 520 provided therein defining a pair of opposed depending arms 522 and 524 for accommodating pivotal movement of the articulating section 412 relative to endoscopic portion 404. Apertures 526 and 528 are respectively provided in the opposed depending arms 522 and 524, and a tail portion 530 extends proximally from the yoke portion 518 thereof with a circumferential groove 532 provided therein. A tang 534 is formed adjacent the distal end of the outer tubular portion 490 for lockingly engaging the circumferential groove 532 in tail portion 530 of yoke portion 518 when it is extended into the axial bore 492 thereof to connect the distal end portion 419 to the remainder of endoscopic portion 404. In addition, an axial passageway of at least the distal portion 515 of central control road 514.

Surgical instrument 400 further comprises a linkage mechanism for moving the articulating section 412 of endoscopic portion 404 within a defined angular degree of rotation. The linkage mechanism includes a base link 540 having a body portion 542 and a tail portion defined by an intermediate circumferential groove section 544 and a proximal annular section 546. Base link 540 is secured within the axial passageway 502 of inner tubular portion 500 by a tang 545 formed adjacent the distal end of inner tubular portion 500, and configured for engaging the circumferential groove section 544 thereof. A central passageway 548 extends through base link 540, adjacent the lower edge thereof, for accommodating a proximal pivot pin 552 which inter-links the base link 540 with a connective link 554 through a proximal aperture 556 provided therein. A distal aperture 558 is also provided in connective link 554 for accommodating distal pivot pin 560 which is received in a corresponding aperture 562 provided in the proximal portion 564 of articulating section 412 adjacent the lower edge thereof, Thus, connective link 554 functions to interlink the articulating section 412 to the base link 540 of the linkage mechanism.

The articulating section, 412 of endoscopic portion 404 has a centrally disposed transverse aperture 566 formed in the proximal portion 564 thereof for accommodating the main pivot pin 414. A longitudinal aperture 570 extends through the articulating section 412 for permitting extension of the central control rod member 514 therethrough. Articulating section 412 further includes opposed depending yoke arms 572 and 574 each having an aligned longitudinal cam slots 576 and 578 formed therein respectively, for cooperating with a cam follower pin 580. In addition, opposed aligned pivot ports 582 and 585 are provided in the lateral yoke arms 572 and 574, respectively.

The co-operating atraumatic retractor rod members 418 and 420 are pivotably connected to the opposed lateral yoke arms 572 and 574 of articulating section 412 by engagement of a pivot pin 586 within the opposed pivot ports 582 and 584, and also within corresponding pivot ports 588 and 590 provided in the retractor rod members 418 and 420, respectively. Symmetrically disposed angular camming slots 592 and 594 are also formed in retractor rod member 418 and 420 respectively, for working with cam follower pin 580. A transverse bore 596 is provided in pivot pin 586 for permitting of a portion of the distal end 15 of central control rod 514 therethrough, and a transverse bore 598 is provided in camming pin 580 for receivable engaging a distal end portion of central control rod 514.

The termination of the distal portion 515 of central control rod 514 within camming pin 580 achieves complete connectivity between the driving assembly in handle portion 402 and the retractor assembly 416 of endoscopic portion 404. Moreover, axial movements of the central control rod 514, in response to axial translation of driving screw 440, will cause corresponding cooperative movement of the retractor rod members 418 and 420 through the translation of camming pin 580 relative to the angular cam slots 592 and 594.

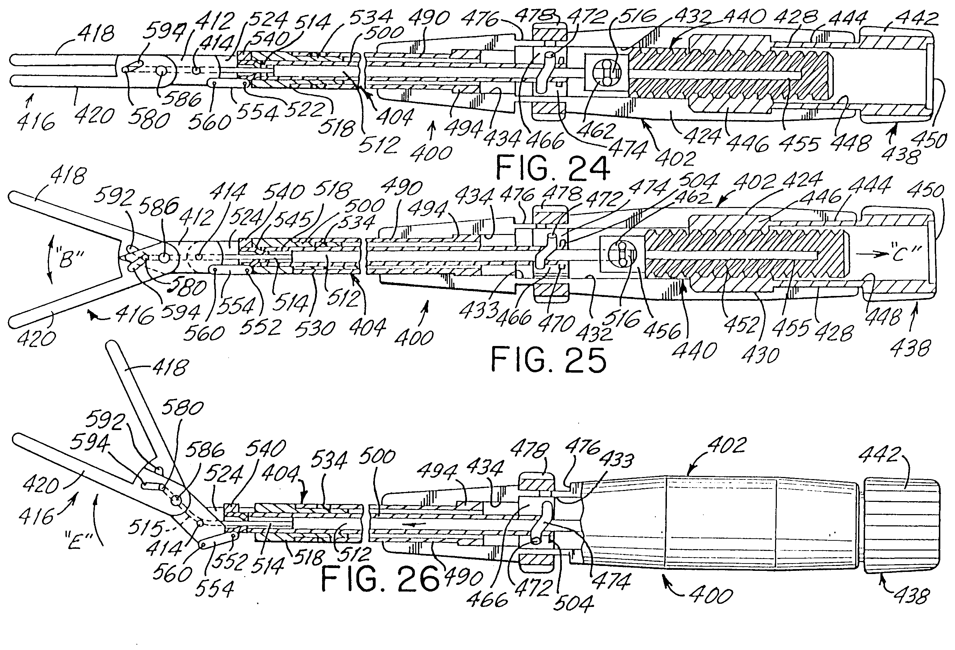

Referring now to Figs. 24-26, the surgical instrument 400 may advantageously be employed as a retractor during gynecological procedures by rotating the knob member 438 counterclockwise (as viewed from the proximal end of the instrument) to move the co-operative retractor rod members 418 and 420 from the closed position of Fig. 24, to the opened position of Fig. 25. More particularly, upon rotating knob member 438 counterclockwise, the drive screw member 440 will translate in the direction of arrow "C". As screw member 440 retreats, the tail portion 516 of the central control rod 514 is pulled in a proximal direction causing the camming pin 580, which is fixed to the distal end thereof, to move proximally within the angled camming slots 592 and 594 of rod members 418 and 420. Distal movement of camming pin 580 within camming slots 592 and 594 causes the co-operating retractor rod members 418 and 420 to open in the direction of arrow "B". Closure of rod members 418 and 420 is achieved through counter-rotation of knob member 438. Transposition of the rotation from counter-clockwise to clockwise for purposes of deploying the retractor assembly is also with the scope of the invention.

Turning now to Fig. 26, in operation, the articulating section 412 of endoscopic portion 404 may be pivoted in the direction of arrow "E" within a vertical plane with respect to the longitudinal axis of endoscopic portion 404 into various angularly disposed positions. To achieve this articulated movement, the manipulating collar 476, 478 is rotated clockwise (as viewed from the proximal end of the instrument), causing cylindrical cam follower 466, 468 to rotate concomitantly therewith. As a cam follower 466, 468 rotates, the cam post 472 translates within the cam slot 474 from a first position, best seen in Fig. 24, wherein collar 478 is in its proximal-most position within circumferential groove 476, to a second position, best seen in Fig. 25, wherein collar 478 is in its distal-most position within groove 476. The axial translation of cylindrical cam follower 464, 466 causes the inner tubular portion 500 of endoscopic portion 404 to move in a distal direction, since the proximal flange 504 of inner tubular portion 500 is engaged in the groove 471 defined in passageway 470 of cam follower 464, 466. As inner tubular portion 504 moves distally, it extends outwardly from the axial bore 535 in the sleeve member defined by the distal portion 410 of endoscopic portion 404, such that base link 540 is urged distally within the slotted area 520 of distal portion 410. Thereupon, connective link 554 is urged in a generally distal direction, pivoting about pivot pins 552 and 560, and causing the articulating section 412 to be pivoted angularly with respect to the longitudinal axis of endoscopic portion 404 about the main pivot pin 414.

The angle of vertical translation of articulating section 412 can vary depending upon the degree of rotation of manipulating collar 478. In addition, once in an articulated position, the retractor assembly 416 can be manipulated independently, since the distal end portion 515 of the central control rod 514 is substantially flexible, as seen in Fig. 26. Transposition of the rotation from clockwise to counter-clockwise for articulating the instrument is also within the scope of the invention.

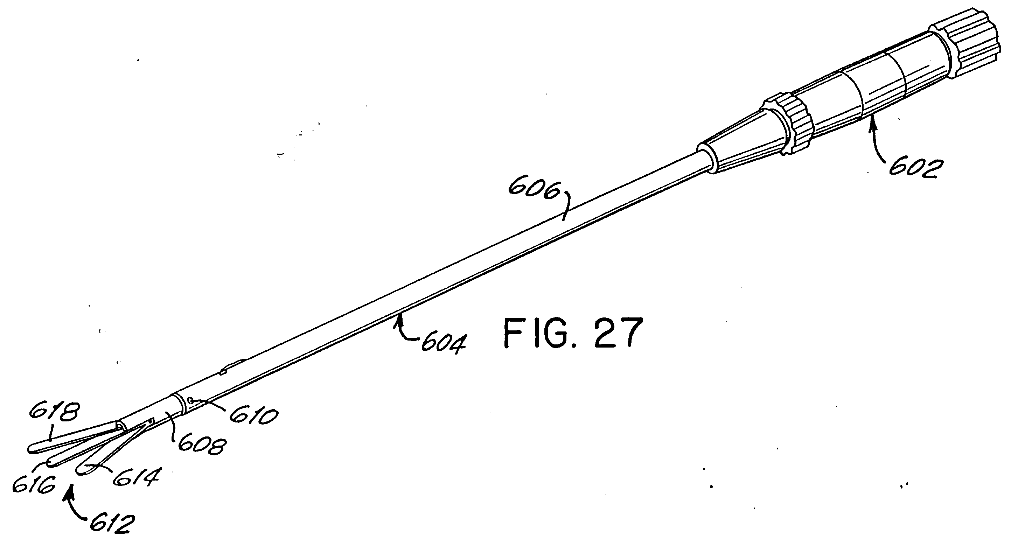

Turning to Fig. 27, yet another embodiment 600 of the articulating surgical retractor of the subject invention is illustrated which may be used endoscopically or during laparoscopic procedures within the abdominal cavity. Surgical instrument 600 comprises an axial handle portion 602, and an elongated endoscopic portion 604 extending from the axial handle portion 602 and including an elongated tubular section 606, and an articulating distal section 608 pivotably connected to the elongated tubular section 606 adjacent the distal end thereof by a main pivot pin 610. A retractor assembly 612 is operatively associated with the articulating section 608 and includes a plurality co-operative interleaved retractor blade members 614, 616, and 618.

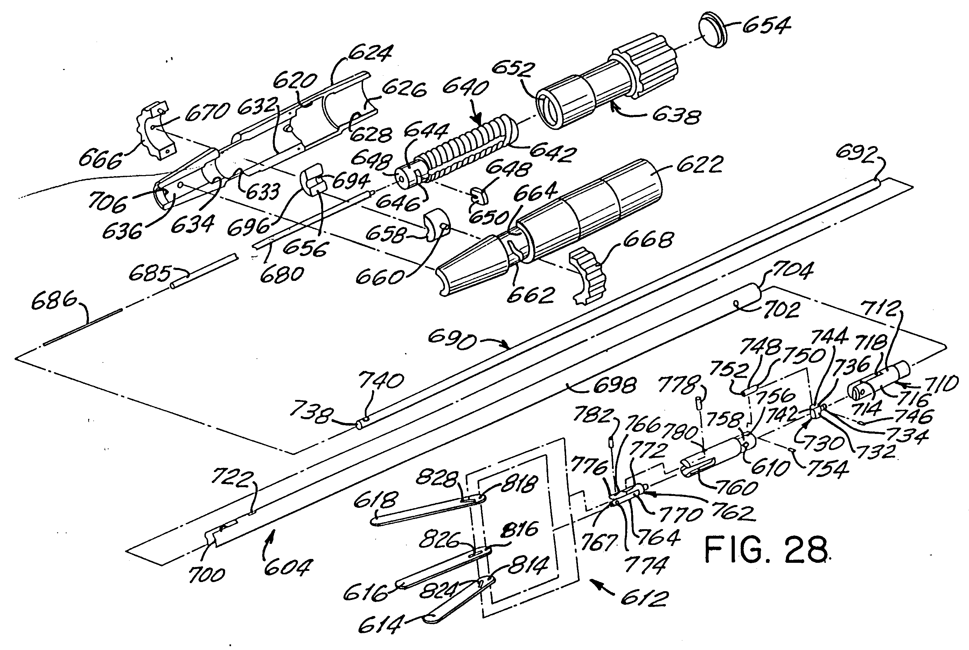

Referring to Fig. 28, the handle portion 602 of surgical instrument 600 is substantially identical to that of surgical instrument 400. It comprises mountable right and left hemi-sections 622 and 624 having a stepped axial bore 626 extending therethrough defined by a proximal chamber 628, a primary medial chamber 630, a secondary medial chamber 632, a tertiary medical chamber 633, a quaternary medial chamber 634, and a distal chamber 26. The handle portion 602 houses, within the axial bore 626, a driving assembly for manipulating the retractor assembly and a camming assembly for manipulating the articulating section 608 of endoscopic portion, 604.

The driving assembly includes a rotatable knob member 638 and an axially advanceable driving screw 640. Driving screw 640 varies from the driving screw 440 of surgical instrument 400 in that it includes a body portion 642 having a distal head portion 644 provided with a transverse slot 646 for accommodating a transverse planar engaging clip 648. Engaging clip 648 is formed with a retention notch 650 for lockingly retaining an operative element of endoscopic portion 604. The knob member 638 is formed with an internal threaded bore 652 extending at least partially therethrough for operatively engaging the driving member 640, and which is covered by closure member 654.

The camming assembly includes a cylindrical cam follower having mountable right and left hemi-sections 656 and 658 configured for rotational and axial movement within the tertiary medial chamber 633 of axial bore 626. A camming post 660 extends radially outward from the periphery of left hemi-section 658 and is dimensioned for translating within a cam slot 662 formed in a circumferential groove 664 defined in the outer surface of handle portion 602 external from the tertiary medial chamber 633. A rotatable manipulating collar defined by mountable right and left hemi-portions 666 and 668 is configured for being mounted in circumferential groove 664 and includes a port 670 for receivably engaging the cam post 660 so as to interlock the elements of the camming assembly. Rotation of manipulating collar 666, 668 will cause translation of cam post 660 in cam slot 662, and consequent axial translation of the cylindrical cam follower 656, 658 along the axial center line of the handle portion 602 with the tertiary medial chamber 633.

The endoscopic portion 604 of surgical instrument 600 comprises a central control rod 680 having a tail portion 682 which extends into an axial bore 684. in driving screw member 640 to be lockingly engaged therein by the notch 650 in locking clip 648. In doing so, the axial translation of the driving screw 640 will cause corresponding axial movement of the control rod 680. A connective rod 686 is engaged in the distal end 685 of control rod 680 for interconnecting the retractor assembly 612 with the control rod 680. Connective rod 686 is flexible to permit operation of the retractor assembly 612 when the articulating section 608 of endoscopic portion 604 is pivoted into an operative position. Endoscopic portion 604 further comprises an inner tubular portion 690 having an annular flange 692 formed on the proximal end thereof. Flange 692 is dimensioned for engagement in a circumferential groove 694 formed in the axial bore 696 of the cylindrical cam follower 656, 658, such that the inner tubular portion 690 will translate axially when the cylindrical cam follower 656, 658 moves within the tertiary medial chamber 633 of axial bore 626.

The inner tubular portion 690 is disposed within the outer tubular portion 698 which is provided for a longitudinally extending slot 700 in the distal end portion thereof for accommodating the pivotal movements of articulating section 608. A transverse aperture area 702 is defined adjacent the proximal end 704 of the outer tubular portion 698 for receiving a radially inwardly projecting stem 706 disposed in the distal chamber 636 of axial bore 626 in handle portion 602. Engagement of stem 706 in aperture area 702 achieves fixation of the endoscopic portion 604 and the handle portion 602.

A primary yoke member 710 is mountable in the distal end portion of the outer tubular portion 698 which comprises a body portion 712 having a pair of opposed lateral yoke arms 714 and 716 depending therefrom and defining a slotted area 718 therebetween. A circumferential groove 720 is provided in the body portion 712 for being engaged by a locking tang 722 formed adjacent the slotted area 700 in the outer tubular portion 698. Opposed pivot ports, of which 724 is one, are defined in the opposed lateral yoke arms 714 and 716 for accommodating main pivot pin 610 which is formed integral with the articulating section 608 of endoscopic portion 604.

A linkage assembly interlinks the articulating section 608 with the inner tubular portion 690 of endoscopic portion 604. The linkage assembly includes a base link 730 having a body portion 732 from which extends a proximal tail portion 734 provided with a circumferential groove 736. The tail portion 734 is adapted to be extended into the distal end 738 of inner tubular portion 690 and is maintained therein by a locking tang 740 which is engagable in the circumferential groove 736. An axial bore 742 extends through the base link 730 for permitting passage of connective rod 686 therethrough. In addition, an aperture 744 is provided in the body portion 732 of base link 730, adjacent the upper edge thereof, for receiving a proximal pivot pin 746 which interlinks base link 730 with a connective link 748 through a proximal aperture 750 formed therein. Connective link 748 has a distal aperture 752 for receiving a distal pivot pin 754 which is provided for interlinking connective link 748 with the articulating section 608 of endoscopic portion 604 through an aperture 756 provided in the proximal end portion 758 of articulating section 608. Axial translation of the inner tubular portion 690, in response to movement of the camming assembly, will cause the corresponding translation of the base link 730 within the slotted portion 718 of primary yoke member 710, whereby the connective link 748 will move generally axially to cause pivoting movement of articulating section 608 in a vertical plane relative to the longitudinal axis of the endoscopic portion 604 of surgical instrument 600.

The articulating section 608 of endoscopic portion 602 is formed with a slotted area 760 for accommodating the retractor assembly 612 of surgical instrument 600. The retractor assembly 612 includes a secondary yoke member 762 having opposed upper and lower yoke arms 764 nd 766 defining a slotted retractor blade maintaining area 768 therebetween. Opposed longitudinally extending guide slots 770 and 772 are provided in upper and lower yoke arms 764 and 766, respectively, as well as opposed pivot apertures 774 and 776, respectively. A guide pin 778 extends through a maintaining aperture 780 in the articulating section 608, and into the opposed guide slot 770 and 772, while a camming pin 782 extends through the opposed pivot apertures 774 and 776. Guide pin 778 and camming pin 782 both cooperate with the interleaved retractor blades 614, 616, and 618. More particularly, corresponding pivot ports 814, 816 and 818 are provided respectively in the interleaved retractor blade 614, 616, and 618, for receiving guide pin 778. Retractor blades 614, 616 and 618 are made of a suitable surgical material having sufficient strength for the desired retractor function. Such materials include stainless steel, plastics and/or combinations thereof. Camming slots are provided in the retractor blades for working with camming pin 782, and include an angularly disposed camming slot 824 is formed in retractor blade 614, a longitudinally disposed camming slot 826 is formed in retractor blade 616, and an angularly disposed camming slot 828 is formed in retractor blade 618. Camming slots 824 and 828 are arranged in symmetrical relationship, such that translation of the camming pin 782, in response to movements of secondary yoke member 762, will cause a fan-like deployment of the interleaved retractor blades 614, 616, and 618.

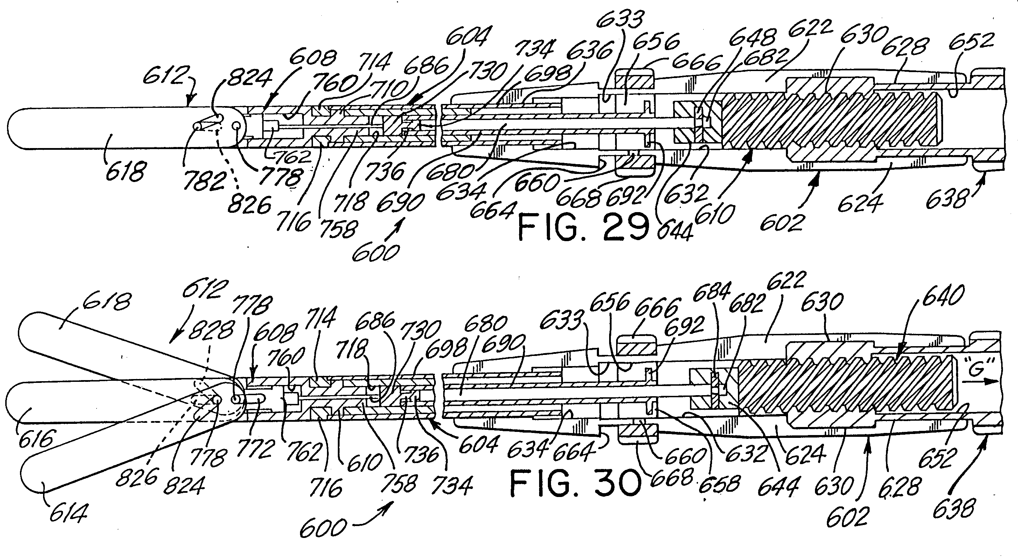

Referring to Figs 29-32, in use, the retractor blade assembly 612 is moved from the closed position illustrated in Fig. 29, to an opened position illustrated in Fig. 30, by rotating the knob member 638 counterclockwise (as viewed from the proximal end of the instrument) to cause corresponding axial translation of screw member 640 in the direction of arrow "G", within the axial bore 626 of handle portion 602. As driving screw member 640 retreats, the tail portion 682 of the central control rod 680 is pulled proximally, along with the connective rod 686, the distal end of which is fixedly mounted in the proximal end of secondary yoke member 762. At such a time, the opposed guide slots 770 and 772 in secondary yoke member 762 permit relative translation of secondary yoke member 762 in relation to the guide pin 778. Furthermore, as the secondary yoke member 762 is drawn in a proximal direction, camming pin 782 translates proximally within the camming slots 824, 826, and 828 of the retractor blades, causing the interleaved retractor blades 614, 616, and 618 to deploy in a fan-like configuration.

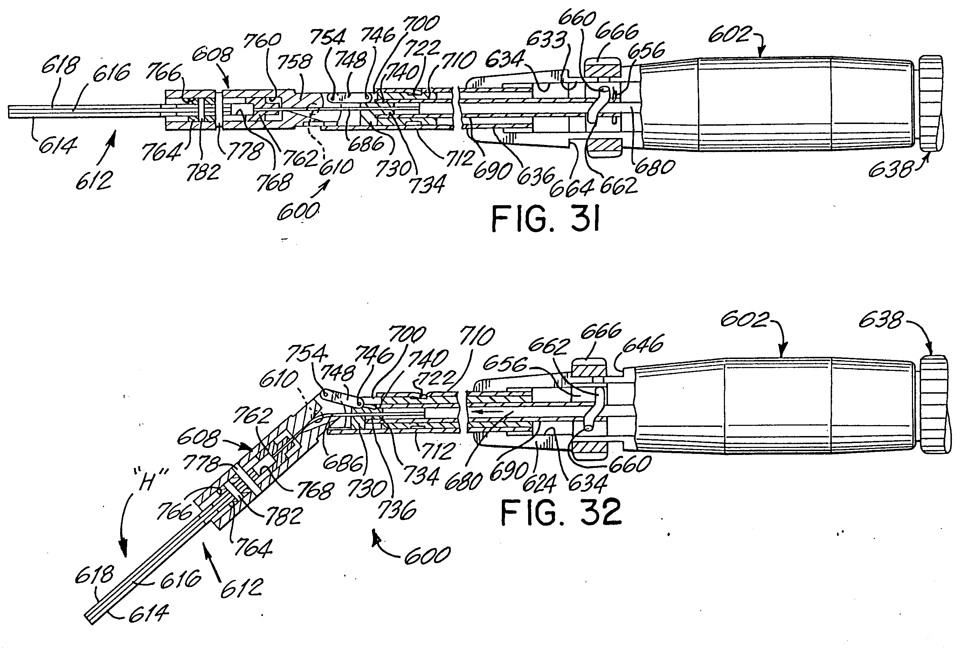

Turning now to Figs. 31 and 32, to pivot the articulating section 608 of endoscopic portions 604 in the direction of arrow "H" in a vertical plane with respect to the longitudinal axis of endoscopic portion 604 during a surgical procedure, the manipulating collar 666, 668 is rotated clockwise (as viewed from the proximal end of the instrument), such that the cylindrical can, follower 656, 658 is caused to advance from a proximal position to a distal position with the tertiary medial chamber 633 of axial bore 626. At the same time, camming post 660 translates within the camming slot 662 formed in the circumferential groove 664 in handle portion 602. Consequently, the inner tubular portion 690 of endoscopic portion 604 moves in a distal direction, urging the base link 730 distally within the slotted area 718 of primary yoke member 710. Thereupon, connective link 748 is urged in a generally distal direction, causing the articulating section 608 to pivot about main pivot pin 610 in a vertical plane relative to the longitudinal axis of endoscopic portion 604. The angle of vertical translation of articulating section 608 can vary depending upon the degree of rotation of the manipulating collar. Furthermore, while the articulating section 608 is in an angular position, the retractor assembly may be actuated independently, since the connective rod 686 is substantially flexible, as seen in Fig. 32.

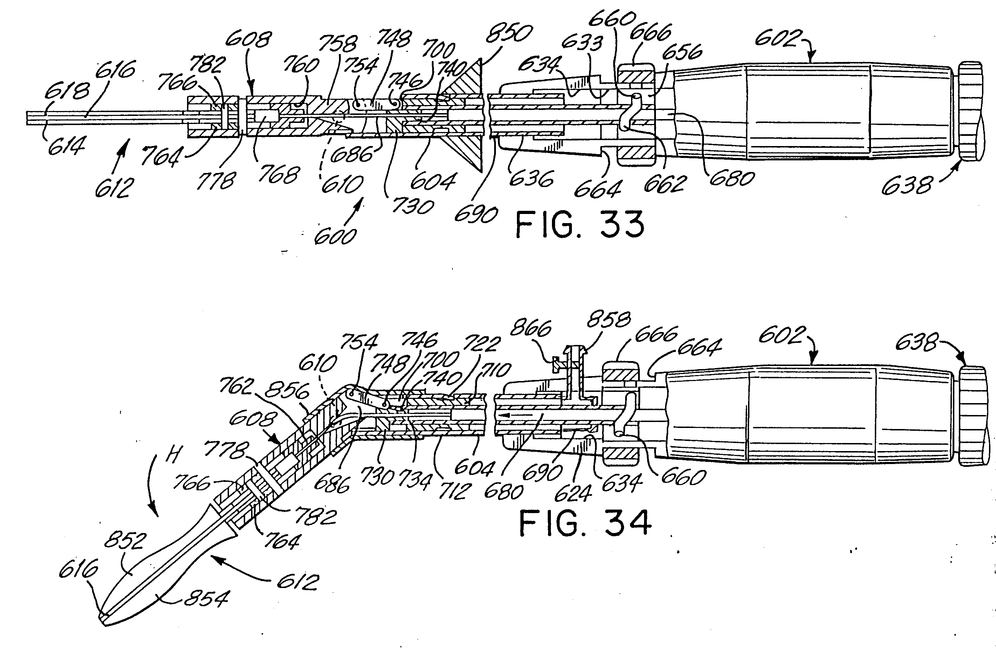

Fig. 33 shows an endoscopic surgical retractor 600 substantially the same as that shown in Figs. 27-32. This retractor 600 is particularly adapted for use in gynecological surgery involving the cervix or uterus. An annular cervical seal 850 is removably disposed on endoscopic portion 604 intermediate the axial handle portion 602 and the articulating distal section 608. This cervical seal 850 assists in preventing the egress of insufflation gases lion the uterus during retractor manipulations.

In use, the uterus is insufflated and the retractor is inserted to a point wherein the cervical seal 850 is adjacent the cervix of the patient. In this position the cervical seal 850 inhibits the flow of insufflation gas from the uterus around the exterior of the endoscopic portion 604 of the instrument 600.

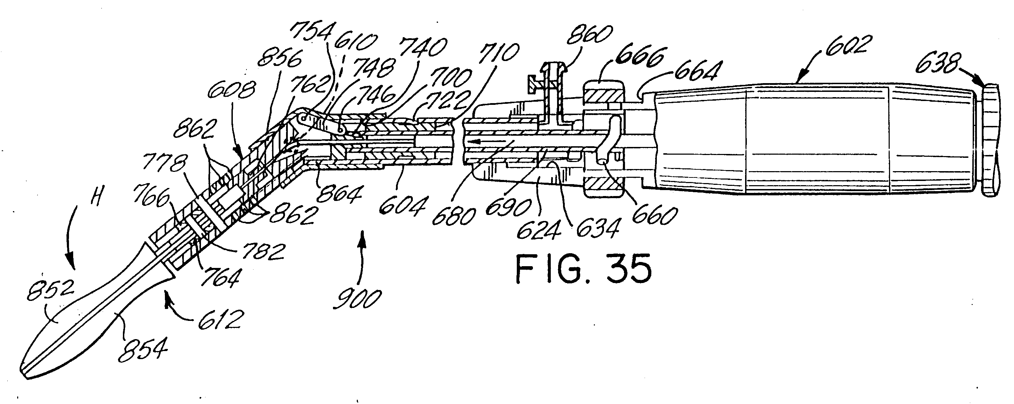

Figs. 34 and 35 show two alternative embodiments of the endoscopic surgical retractor in accordance with the present invention. These retractors are specifically designed and adapted for gynecological applications and include atraumatic upper and lower blades 852, 854 configured in a streamlined semicircular cross section. In the retracted configuration, the blades interleave to form a smooth rounded retractor assembly for atraumatic insertion, particularly when used without a cannula port.

The endoscopic surgical retractors shown in Figs. 34 and 35 are also provided with a sleeve 856 extending around the distal end of the endoscopic portion 604 and the proximal end of the articulating portion 608. This sleeve 856 serves to enclose and protect the articulating linkages from external contamination. Also, the sleeve 856 prevents tissue or organs from becoming entangled in the linkages during operation. The sleeve 856 is preferably formed of an elastic or silastic material capable of moving with the articulating portion 608.

Referring specifically to Fig. 34, the endoscopic surgical retractor includes an injection port 858 disposed adjacent a distal end of the handle portion 602. The injection port 858 communicates with the interior of the endoscopic portion 604 to permit fluid to be injected therethrough into the surgical site. The injection port 858 of the retractor in Fig. 34 comprises a substantially inverted "T" structure providing direct access to the passages in the endoscopic portion 604. This structure may advantageously be used for fluid irrigation or medication of the operative site. Alternatively, the port may be used to administer dyes or marker substances intravaginally such as, for example, radiopaque dyes injected to determine the patency of the fallopian tubes, etc. Where a surgical retractor having an injection port feature is to be used in an insufflated cavity, valve 866 is positioned in line to inhibit insufflation gases from exiting the cavity through the port.

The endoscopic surgical retractor of Fig. 35 is substantially the same as the retractor of Fig. 34 except that the injection port 860 communicates with a plurality of distribution ports 862 through tube 864 disposed coaxially in endoscopic portion 604. This configuration allows for more accurate delivery of fluids and may even be used to administer pressurized aerosols therethrough. The endoscopic surgical instrument of the subject invention is compact, lightweight and easy to use. It is intended to enable the surgeon to use the instrument with one hand, thus freeing the other hand for performance of other surgical tasks.

The claims which follow below define embodiments of the invention additional to those described in detail above.

## Claims

1. A surgical apparatus (400, 600) comprising:
an endoscopic portion (404, 604) including an elongate tubular section having opposed proximal and distal ends;
an articulating section (412, 608) pivotally connected to a distal end portion of said endoscopic portion;
tool means (418, 420, 612) operatively associated with said articulating section and including at least two co-operating members (418, 420, 614, 616, 618) movable between a closed position and an open position; and
means (480, 680) co-operating with a proximal portion of said endoscopic portion for pivoting said articulating section relative to a longitudinal axis of said endoscopic portion within an angular degree of rotation; and characterised by
rotating means (438, 638) co-operating with a proximal end of said endoscopic portion for deploying said at least two co-operating members into a variety of intermediate positions between said closed position and said opened position.

2. Apparatus as claimed in claim 1 wherein said pivoting means includes knob means (438) mounted for rotation relative to said endoscopic portion and driving means (440) threadably associated with said knob means movable in an axial direction in response to rotation of said knob means.

3. Apparatus as claimed in claim 2, wherein said knob means comprises an elongated knob member (438) having a threaded axial bore extending at least partially therethrough for operatively receiving said driving means.

4. Apparatus as claimed in claim 2 or 3, wherein said driving means comprises an elongated threaded screw member (440), and an elongated control rod (514) extending from a distal end of said screw member to said retractor assembly.

5. Apparatus as claimed in any one of the preceding claims, wherein said means for pivoting said articulating section includes camming means (464, 466, 474) associated with said handle portion and cooperative linkage means associated with said endoscopic portion.

6. Apparatus as claimed in claim 5, wherein said camming means including a barrel cam assembly (464, 466) including a cam slot (474) defined in said handle portion and a cam follower (472) disposed in said handle portion for translating relative to said cam slot between a proximal position and a distal position.

7. Apparatus as claimed in claim 6, wherein said linkage means includes an elongate inner tubular member (500) having a proximal end portion associated with said cam follower and a distal end portion associated with a linkage mechanism interconnecting said articulating section to said camming means.

8. Apparatus as claimed in claim 7, wherein said linkage mechanism includes a base link (540) connected to said distal end portion of said inner tubular member and a connective link (554) pivotably connected to said base link at one end thereof and to said articulating section at the opposed end thereof.

9. Apparatus as claimed in any one of the preceding claims, and further comprising:
fluid delivery means (858), integral with said surgical apparatus, for passing fluid through said endoscopic portion to an operative site.

10. Apparatus as claimed in claim 9 wherein said fluid delivery means includes an injection port (858) disposed in said handle portion and communicating with the interior of said endoscopic portion.

11. Apparatus as claimed in claim 10 wherein said injection port is connected to at least one distribution port by a connective tube (864).

12. Apparatus as claimed in any one of the preceding claims, further comprising sleeve means (856) for protecting the pivotal connection between the articulating section and the endoscopic portion.

13. Apparatus as claimed in claim 12 wherein said sleeve means is comprised of a flexible plastic material, said flexible plastic material being sealed to both the articulating section and the distal end of the endoscopic portion.

14. Apparatus as claimed in claim 9,10,11 or 12 wherein said at least two cooperating members are grasping jaws or dissecting jaws.

15. Apparatus as claimed in any one of the preceding claims wherein said tool means comprises a retractor assembly (216).

16. Apparatus as claimed in claim 15 wherein said retractor assembly comprises a pair of atraumatic cooperating rod members (852, 854).

17. Apparatus as claimed in claim 15 wherein said retractor assembly includes a plurality of interleaved blade members (614, 616, 618).

18. Apparatus as claimed in claim 17 wherein said interleaved blade members are collectively formed in a substantially cylindrical shape having an atraumatic end portion when in the closed configuration.

19. Apparatus as claimed in any one of claims 15 to 18, wherein said retractor assembly further includes a yoke member (762) having a set of cam slots (770, 772), and a camming pin (778) movable within said of cam slots for cooperating with said members.

## Patentansprüche

1. Chirurgische Vorrichtung (400, 600), umfassend:
einen endoskopischen Bereich (404, 604) umfassend einen langgestreckten röhrenförmigen Abschnitt mit entgegengesetzten proximalen und distalen Enden;
einen Gelenkabschnitt (412, 608), der schwenkbar mit einem distalen Endbereich des endoskopischen Bereichs verbunden ist;
eine Werkzeugeinrichtung (418, 420, 612), die betriebsmäßig dem Gelenkabschnitt zugeordnet ist und zumindest zwei zusammenwirkende Elemente (418, 420, 614, 616, 618) aufweist, die zwischen einer geschlossenen Position und einer offenen Position bewegbar sind; und
eine Einrichtung (480, 680), die mit einem proximalen Endbereich des endoskopischen Bereichs zusammenwirkt, um den Gelenkabschnitt relativ zu einer Längsachse des endoskopischen Bereichs innerhalb eines Drehtwinkelmaßes zu verschwenken; und gekennzeichnet durch
eine Dreheinrichtung (438, 638), die mit einem proximalen Ende des endoskopischen Bereichs zusammenwirkt, um die zumindest zwei zusammenwirkenden Elemente in einer Vielzahl von Zwischenpositionen zwischen der geschlossenen Position und der geöffneten Position auszufahren.

2. Vorrichtung gemäß Anspruch 1, wobei die Schwenkeinrichtung eine Knopfeinrichtung (438) aufweist, die zu einer Drehung relativ zum endoskopischen Bereich montiert ist, und eine Antriebseinrichtung (440), die durch ein Gewinde der Knopfeinrichtung zugeordnet ist und in einer axialen Richtung in Antwort auf eine Drehung der Knopfeinrichtung bewegbar ist.

3. Vorrichtung gemäß Anspruch 2, wobei die Knopfeinrichtung ein langgestrecktes Knopfelement (438) umfaßt, mit einer axialen Gewindebohrung, die sich zumindest teilweise durch dieses hindurch erstreckt, um betriebsmäßig die Antriebseinrichtung aufzunehmen.

4. Vorrichtung gemäß Anspruch 2 oder 3, wobei die Antriebseinrichtung ein langgestrecktes, mit Gewinde versehenes Schraubenelement (440) umfaßt und eine langgestreckte Kontrollstange (514), die sich von einem distalen Ende des Schraubenelementes zu dem Retraktoraufbau erstreckt.

5. Vorrichtung gemäß einem der vorhergehenden Ansprüche, wobei die Einrichtung zum Verschwenken des Gelenkabschnittes eine Nockeneinrichtung (464, 466, 474), die dem Griffbereich zugeordnet ist, und eine zusammenwirkende Verbindungseinrichtung, die dem endoskopischen Bereich zugeordnet ist, aufweist.

6. Vorrichtung gemäß Anspruch 5, wobei die Nockeneinrichtung einen Trommelnockenaufbau (464, 466) einschließlich einem Nockenschlitz (474) aufweist, der in dem Griffbereich begrenzt ist, und einen Nockenfolger (472), der in dem Griffbereich angeordnet ist, um sich relativ zu dem Nockenschlitz zwischen einer proximalen Position und einer distalen Position zu verschieben.

7. Vorrichtung gemäß Anspruch 6, wobei die Verbindungseinrichtung ein langgestrecktes, inneres röhrenförmiges Element (500) aufweist mit einem proximalen Endbereich, der dem Nockenfolger zugeordnet ist, und einem distalen Endbereich, der einen Verbindungsmechanismus, der den Gelenkabschnitt mit der Nockeneinrichtung verbindet, zugeordnet ist.

8. Vorrichtung gemäß Anspruch 7, wobei der Verbindungsmechanismus eine Grundverbindung (540) aufweist, die mit dem distalen Endbereich des inneren röhrenförmigen Elementes verbunden ist und ein Verbindungsgelenk (554), das schwenkbar mit der Grundverbindung an einem Ende derselben und dem Gelenkabschnitt an dem entgegengesetzten Ende derselben schwenkbar verbunden ist.

9. Vorrichtung gemäß einem der vorhergehenden Ansprüche, weiter umfassend: eine Fluidausgabeeinrichtung (858) einstückig mit der chirurgischen Vorrichtung, um Fluid durch den endoskopischen Bereich zum Operationsort hindurchzuführen.

10. Vorrichtung gemäß Anspruch 9, wobei die Fluidausgabeeinrichtung eine Injektionsöffnung (858) aufweist, die in dem Griffbereich angeordnet ist und mit dem inneren endoskopischen Bereich in Verbindung steht.

11. Vorrichtung gemäß Anspruch 10, wobei die Injektionsöffnung mit zumindest einer Verteilöffnung durch eine Verbindungsröhre (864) verbunden ist.

12. Vorrichtung gemäß einem der vorhergehenden Ansprüche, weiter umfassend eine Hülseneinrichtung (856), um die Schwenkverbindung zwischen dem Gelenkabschnitt und dem endoskopischen Bereich zu schützen.

13. Vorrichtung gemäß Anspruch 12, wobei die Hülseneinrichtung aus einem biegsamen Kunststoffmaterial besteht und das biegsame Kunststoffmaterial sowohl am Gelenkabschnitt als auch am distalen Ende des endoskopischen Bereichs abgedichtet ist.

14. Vorrichtung gemäß Anspruch 9, 10, 11 und 12, wobei die zumindest zwei zusammenwirkenden Elemente Greifklauen oder Dissektorklauen sind.

15. Vorrichtung gemäß einem der vorhergehenden Ansprüche, wobei die Werkzeugeinrichtung einen Retraktoraufbau (216) umfaßt.

16. Vorrichtung gemäß Anspruch 15, wobei der Retraktoraufbau ein Paar von atraumatischen, zusammenwirkenden Stangenelementen (852, 854) umfaßt.

17. Vorrichtung gemäß Anspruch 15, wobei der Retraktoraufbau eine Mehrzahl von überlappenden Blattelementen (614, 616, 618) aufweist.

18. Vorrichtung gemäß Anspruch 17, wobei die überlappenden Blattelemente gemeinsam in einer im wesentlichen zylinderförmigen Form mit einem atraumatischen Endbereich in der geschlossenen Gestalt gebildet sind.

19. Vorrichtung gemäß einem der Ansprüche 15 bis 18, wobei der Retraktoraufbau weiterhin ein Jochelement (762) mit einem Satz von Nockenschlitzen (770, 772) aufweist, und ein Nockenstift (778) innerhalb der Nockenschlitze zum Zusammenwirken mit den Elementen bewegbar ist.

## Revendications

1. Appareil chirurgical (400, 600) comprenant :
une portion endoscopique (404, 604) incluant une section tubulaire oblongue ayant des extrémités proximale et distale opposées ;
une section d'articulation (412, 608) reliée d'une manière pivotante à une portion d'extrémité distale de ladite portion endoscopique ;
un moyen d'outil (418, 420, 612) fonctionnellement associé à ladite section d'articulation et incluant au moins deux éléments de coopération (418, 420, 614, 616, 618) déplaçables entre une position fermée et une position ouverte ; et
un moyen (480, 680) coopérant avec une portion proximale de ladite portion endoscopique pour faire pivoter ladite section d'articulation relativement à un axe longitudinal de ladite portion endoscopique à l'intérieur d'un degré de rotation angulaire ; et caractérisé par
un moyen de rotation (438, 638) coopérant avec une extrémité proximale de ladite portion endoscopique pour déployer lesdits au moins deux éléments de coopération en une variété de positions intermédiaires entre ladite position fermée et ladite position ouverte.

2. Appareil selon la revendication 1, où ledit moyen de pivotement comporte un moyen de bouton (438) installé à rotation relativement à ladite portion endoscopique et un moyen d'entraînement (440) associé par filetage audit moyen de bouton déplaçable dans une direction axiale en réponse à la rotation dudit moyen de bouton.

3. Appareil selon la revendication 2, où ledit moyen de bouton comprend un élément de bouton oblong (438) ayant un perçage axial fileté s'étendant au moins partiellement à travers celui-ci pour recevoir fonctionnellement ledit moyen d'entraînement.

4. Appareil selon la revendication 2 ou 3, où ledit moyen d'entraînement comprend un élément de vis filetée oblongue (440) et une tige de commande oblongue (514) s'étendant d'une extrémité distale dudit élément de vis audit ensemble d'écarteur.

5. Appareil selon l'une des revendications précédentes, où ledit moyen pour faire pivoter ladite section d'articulation comporte un moyen de came (464, 466, 474) associé à ladite portion de poignée et un moyen de liaison coopérant associé à ladite portion endoscopique.

6. Appareil selon la revendication 5, où ledit moyen de came comprend un ensemble de came périphérique (464, 466) incluant une fente de came (474) définie dans ladite portion de poignée et un suiveur de came (472) disposé dans ladite portion de poignée pour translater relativement à ladite fente de came entre une position proximale et une position distale.

7. Appareil selon la revendication 6, où ledit moyen de liaison comporte un élément tubulaire interne oblong (500) ayant une portion d'extrémité proximale associée audit suiveur de came et une portion d'extrémité distale associée à un mécanisme de liaison interconnectant ladite section d'articulation audit moyen de came.

8. Appareil selon la revendication 7, où ledit mécanisme de liaison comporte une pièce de liaison de base (540) reliée à ladite portion d'extrémité distale dudit élément tubulaire interne et une belle de connexion (554) reliée d'une manière pivotante à ladite pièce de liaison de base à une extrémité de celle-ci et à ladite section d'articulation à l'extrémité opposée de celle-ci.

9. Appareil selon l'une des revendications précédentes, et comprenant en outre : un moyen de transmission de fluide (858) intégral avec ledit appareil chirurgical pour faire passer du fluide à travers ladite portion endoscopique à un site opératoire.

10. Appareil selon la revendication 9, où ledit moyen de transmission de fluide comporte un orifice d'injection (858) disposé dans ladite portion de poignée et communiquant avec l'intérieur de ladite portion endoscopique.

11. Appareil selon la revendication 10, où ledit orifice d'injection est relié à au moins un orifice de distribution par un tube de connexion (864).

12. Appareil selon l'une des revendications précédentes, comprenant en outre un moyen formant manchon (856) pour protéger la connexion pivotante entre la section d'articulation et la portion endoscopique.

13. Appareil selon la revendication 12, où ledit moyen formant manchon est constitué d'une matière plastique flexible, ladite matière plastique flexible étant scellée à la fois à la section d'articulation et à l'extrémité distale de la portion endoscopique.

14. Appareil selon la revendication 9, 10, 11 ou 12, où lesdits au moins deux éléments de coopération sont des mâchoires de préhension ou des mâchoires de dissection.

15. Appareil selon l'une des revendications précédentes, où ledit moyen d'outil comprend un ensemble d'écarteur (216).

16. Appareil selon la revendication 15, où ledit ensemble d'écarteur comprend une paire d'éléments de tige atraumatiques coopérants (852, 854).

17. Appareil selon la revendication 15, où ledit ensemble d'écarteur comporte une pluralité d'éléments de lame entrelacés (614, 616, 618).

18. Appareil selon la revendication 17, où lesdits éléments de lame entrelacés sont réalisés collectivement selon une forme sensiblement cylindrique ayant une portion d'extrémité atraumatique lorsqu'ils se trouvent dans la configuration fermée.

19. Appareil selon l'une des revendications 15 à 18, où ledit ensemble d'écarteur comporte en outre un élément d'étrier (762) ayant un ensemble de fentes de came (770, 772) et un axe de came (778) déplaçable dans lesdites fentes de came pour coopérer avec lesdits éléments.
